(19) Europäisches Patentamt

European Patent Office

Office européen des brevets

(11) **EP 4 410 971 A1**

(12) **EUROPEAN PATENT APPLICATION**
published in accordance with Art. 153(4) EPC

(43) Date of publication:
**07.08.2024 Bulletin 2024/32**

(21) Application number: **22876533.5**

(22) Date of filing: **30.09.2022**

(51) International Patent Classification (IPC):
*C12N 7/01* (2006.01)  *C12N 5/10* (2006.01)
*C12N 15/86* (2006.01)  *C12N 15/864* (2006.01)

(52) Cooperative Patent Classification (CPC):
**C12N 5/10; C12N 7/00; C12N 15/86; C12N 15/864**

(86) International application number:
**PCT/JP2022/036684**

(87) International publication number:
**WO 2023/054671 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.09.2021  JP 2021160729**

(71) Applicant: **FUJIFILM Corporation
Tokyo 106-8620 (JP)**

(72) Inventors:
• **MORI Yusuke
Ashigarakami-gun, Kanagawa 258-8577 (JP)**

• **UMETANI Sayako
Ashigarakami-gun, Kanagawa 258-8577 (JP)**
• **MURAGUCHI Taichi
Ashigarakami-gun, Kanagawa 258-8577 (JP)**

(74) Representative: **Hoffmann Eitle
Patent- und Rechtsanwälte PartmbB
Arabellastraße 30
81925 München (DE)**

Remarks:
The complete document including Reference
Table(s) and the Sequence Listing(s) can be
downloaded from the EPO website

(54) **METHOD FOR PRODUCING ADENO-ASSOCIATED VIRUS, CELLS AND EXPRESSION VECTOR**

(57)    An object of the present invention is to provide a manufacturing method of an adeno-associated virus, which can improve a production amount of the adeno-associated virus, and to provide a cell and an expression vector for use in the manufacturing method of an adeno-associated virus. According to the present invention, provided is a manufacturing method of an adeno-associated virus, including expressing an E4ORF6 gene with an exogenous promoter, in which expression of E4ORF6 is reduced as compared with a case where E4ORF6 is expressed using an expression unit containing an enhancer, a cytomegalovirus-derived promoter, a Kozak sequence, an E4ORF6 gene, and a polyA signal sequence.

EP 4 410 971 A1

**Description**

Technical Field

The present invention relates to a manufacturing method of an adeno-associated virus. Furthermore, the present invention relates to a cell and an expression vector for use in the manufacturing method of an adeno-associated virus.

Background Art

The adeno-associated virus (also referred to as AAV) is a linear single-stranded DNA virus belonging to Parvoviridae. The wild-type AAV genome contains a replication regulatory gene (Rep gene) and a capsid structural gene (Cap gene), and inverted terminal repeat (ITR) sequences are adjacent to these genes for viral replication and packaging. The AAV vector can transfer a gene to proliferating and non-proliferating cells, and can be expressed for a long term particularly in non-dividing cells. In addition, AAV is considered to be non-pathogenic and has low immunogenicity. From the above, the AAV vector has been clinically applied as a vector for gene therapy.

AAV is a non-enveloped virus that proliferates in the presence of helper viruses such as adenovirus and herpesvirus. In the preparation of AAV to be used for gene therapy or nucleic acid transfer, classically, AAV replication has been performed by co-infecting host cells with an adenovirus. In addition, a gene responsible for the helper action of adenovirus has been clarified, and a plasmid bearing this gene has also been used. For example, a plasmid containing a Rep gene and a Cap gene, an adenovirus helper plasmid, and a plasmid containing a transgene are simultaneously transfected into HEK293 cells, and can thus be packaged as recombinant AAV (rAAV).

Patent Document 1 describes (a) a base sequence encoding a REP protein of AAV, (b) a base sequence encoding a capsid structural protein of AAV, (c) a base sequence containing a first inverted terminal repeat (ITR) of AAV, (d) a base sequence containing a second inverted terminal repeat (ITR) of AAV, (e) a base sequence for inserting a base sequence encoding a foreign protein and/or a base sequence encoding a foreign protein located between the first ITR and the second ITR, (f) a base sequence containing an E2A region of an adenovirus, (g) a base sequence containing an E4 region of an adenovirus, and (h) a nucleic acid molecule including a base sequence containing a VA-RNA region of an adenovirus.

Patent Document 2 describes a mammalian cell including at least four distinct recombinant target sites (RTS), an adenovirus (Ad) gene including E1A, E1B, or a combination thereof, and a promoter operably linked to the Ad gene, in which the RTS, the Ad gene, and the promoter are chromosomally-integrated; a method of using the cell for generating a recombinant adeno-associated virus (rAAV) producer host cell; and a method of using the AAV producer host cell to produce, package, and purify rAAV.

Patent Document 3 describes a two-plasmid system including a helper plasmid and a vector plasmid, in which the helper plasmid contains at least one Rep gene encoding at least one functional Rep protein and does not contain a Cap gene encoding a functional set of Cap proteins.

Patent Document 4 describes an in vitro method of producing a recombinant AAV virion in a mammalian host cell, the method comprising incubating the cell in a transfection medium, the cell containing (i) an accessory construct including a helper plasmid containing adenovirus E2, E4ORF6, and VA-RNA genes, (ii) an AAV helper construct including a transplasmid containing AAV Rep and AAV Cap operably linked to one or more regulatory sequences, and (iii) an AAV vector including a plasmid (pCis) containing an AAV inverted terminal repeat sequence flanking a target heterologous gene operably linked to one or more regulatory sequences.

Prior Art Documents

Patent Documents

   Patent Document 1: JP2020-188763A
   Patent Document 2: JP2020-507331A
   Patent Document 3: WO2020/208379A
   Patent Document 4: US2016/0222356A

**SUMMARY OF THE INVENTION**

In the above-described related art, the E4 gene region is encoded in the helper plasmid, but the optimization of the expression level of the E4 gene has not been studied. An object of the present invention is to provide a manufacturing method of an adeno-associated virus, by which the production amount of the adeno-associated virus can be improved. Another object of the present invention is to provide a cell and an expression vector for use in the manufacturing method of an adeno-associated virus.

As a result of intensive studies to solve the above-described problems, the present inventors have found that the production amount of the adeno-associated virus can be improved by reducing the expression level of E4ORF6, which is a gene necessary for the production of the adeno-associated virus, or by reducing the amount of the E4ORF6 gene transferred into the cells. The present invention has been completed based on the above findings.

According to an aspect of the present invention, the following inventions are provided.

<1> A manufacturing method of an adeno-associated virus, comprising expressing an E4ORF6 gene with an exogenous promoter, in which expression of E4ORF6 is reduced as compared with a case where E4ORF6 is expressed using an expression unit containing an enhancer, a cytomegalovirus-derived promoter, a Kozak sequence, an E4ORF6 gene, and a polyA signal sequence.

<2> The manufacturing method of an adeno-associated virus according to <1>, in which the reduction of the expression of E4ORF6 is reduction of mRNA expression of E4ORF6 and/or reduction of protein expression of E4ORF6.

<3> The manufacturing method of an adeno-associated virus according to <1> or <2>, in which expression level of E4ORF6 is reduced to 1/2 or less as compared with a case where E4ORF6 is expressed using an expression unit containing an enhancer, a cytomegalovirus-derived promoter, a Kozak sequence, an E4ORF6 gene, and a polyA signal sequence.

<4> The manufacturing method of an adeno-associated virus according to any one of <1> to <3>, in which the expression of E4ORF6 is reduced by any one or more of the following (a) to (e),

    (a) the exogenous promoter is a promoter having a weaker transcriptional activity than the cytomegalovirus-derived promoter,
    (b) the Kozak sequence of the E4ORF6 gene is a modified Kozak sequence, or the Kozak sequence is not used,
    (c) the enhancer sequence is a modified enhancer sequence, or the enhancer sequence is not used,
    (d) the polyA signal sequence is a modified polyA signal sequence, or the polyA signal sequence is not used, and
    (e) the E4ORF6 gene is expressed using an IRES sequence.

<5> The manufacturing method of an adeno-associated virus according to any one of <1> to <4>, in which an amount of the E4ORF6 gene transferred into a cell is 1/2 or less of an amount of one or more genes transferred into a cell among a Rep gene, a Cap gene, an E2A gene, a VA-RNA gene, or a gene for desired therapy or prophylaxis.

<6> A manufacturing method of an adeno-associated virus, comprising transferring an E4 region containing at least an E4ORF6 gene into a cell, in which an amount of the E4ORF6 gene transferred into the cell is 1/2 or less of an amount of one or more genes transferred into the cell among a Rep gene, a Cap gene, an E2A gene, a VA-RNA gene, or a gene for desired therapy or prophylaxis.

<7> The manufacturing method of an adeno-associated virus according to <6>, in which an amount of the E4 region transferred into the cell is 1/10 or less of the amount of the one or more genes transferred into the cell among the Rep gene, the Cap gene, the E2A gene, the VA-RNA gene, or the gene for desired therapy or prophylaxis.

<8> The manufacturing method of an adeno-associated virus according to <6> or <7>, in which a plasmid vector for transferring the E4 region into the cell is different from a plasmid vector for transferring the E2A gene and the VA-RNA gene into the cell.

<9> The manufacturing method of an adeno-associated virus according to any one of <6> to <8>, in which the E4 region consists of only the E4OPF6 gene.

<9A> The manufacturing method of an adeno-associated virus according to any one of <6> to <9>, in which the E4 region is present on a downstream of a promoter capable of regulating expression.

<9B> The manufacturing method of an adeno-associated virus according to any one of <6> to <9> and <9A>, in which the E4 region consists of only the E4ORF6 gene and does not contain a polyA signal sequence.

<10> A cell comprising at least an E4ORF6 gene to be expressed by an exogenous promoter, in which expression of E4ORF6 is reduced as compared with a case where E4ORF6 is expressed using an expression unit containing an enhancer, a cytomegalovirus-derived promoter, a Kozak sequence, an E4ORF6 gene, and a polyA signal sequence.

<11> The cell according to <10>, in which the E4ORF6 gene expressed by the exogenous promoter is transferred into the cell by an expression vector which satisfies any one or more of the following (a) to (e),

    (a) the exogenous promoter is a promoter having a weaker transcriptional activity than the cytomegalovirus-derived promoter,
    (b) the Kozak sequence of the E4ORF6 gene is a modified Kozak sequence, or a Kozak sequence is not provided,
    (c) the enhancer sequence is a modified enhancer sequence, or the enhancer sequence is not provided,
    (d) the polyA signal sequence is a modified polyA signal sequence, or the polyA signal sequence is not provided, and
    (e) the E4ORF6 gene is expressed using an IRES sequence.

<12> A cell comprising at least an E4ORF6 gene to be expressed by an exogenous promoter, in which an amount of the E4ORF6 gene transferred into a cell is lower than an amount of one or more genes transferred into the cell among a Rep gene, a Cap gene, an E2A gene, a VA-RNA gene, or a gene for desired therapy or prophylaxis.

<13> The cell according to any one of <10> to <12>, further comprising an E2A gene and a VA-RNA gene.

<14> The cell according to any one of <10> to <13>, further comprising a Rep gene.

<15> The cell according to any one of <10> to <14>, further comprising a Cap gene and/or a gene for desired therapy or prophylaxis.

<16> An expression vector comprising E4OPF6 gene linked to an exogenous promoter, in which the expression vector satisfies any one or more of the following (a) to (e),

(a) the exogenous promoter is a promoter having a weaker transcriptional activity than the cytomegalovirus-derived promoter,
(b) a Kozak sequence of the E4ORF6 gene is a modified Kozak sequence, or the Kozak sequence is not provided,
(c) an enhancer sequence is a modified enhancer sequence, or the enhancer sequence is not provided,
(d) a polyA signal sequence is a modified polyA signal sequence, or the polyA signal sequence is not provided, and
(e) the E4ORF6 gene is expressed using an IRES sequence.

According to the manufacturing method of an adeno-associated virus, the cell, and the expression vector of the present invention, the production amount of the adeno-associated virus can be improved.

**BRIEF DESCRIPTION OF THE DRAWINGS**

Fig. 1 shows the measurement results of the E4ORF6 expression level.
Fig. 2 shows the measurement results of the AAV genome titer.
Fig. 3 shows measurement results of cytotoxicity.
Fig. 4 shows the measurement results of the E4ORF6 expression level.
Fig. 5 shows the measurement results of the AAV genome titer.
Fig. 6 shows the construct of a plasmid.
Fig. 7 shows the measurement results of the E4ORF6 expression level.
Fig. 8 shows the measurement results of the AAV genome titer.
Fig. 9 shows measurement results of the number of proliferated cells.
Fig. 10 shows the measurement results of the AAV genome titer.

**DESCRIPTION OF THE PREFERRED EMBODIMENTS**

Hereinafter, an example of the embodiment of the present disclosure will be described. However, the present disclosure is not limited to the following embodiments, and can be implemented with appropriate changes within the scope of the object of the present disclosure. In the present specification, a numerical range indicated using "to" means a range including numerical values described before and after "to" as a minimum value and a maximum value, respectively.

<Explanation of terms>

A promoter means a DNA regulatory region/sequence to which an RNA polymerase can be bound and which is involved in the initiation of transcription of a downstream coding sequence or a downstream non-coding sequence.

The vector is a DNA or RNA molecule that is used to artificially transport an exogenous gene to another cell. A vector for expressing a gene is referred to as an expression vector. In a case where a vector containing an exogenous gene to be transferred is transferred into a cell, the exogenous gene is replicated and/or expressed in the cell. Examples of the vector include an episomal (for example, plasmid) vector and a non-episomal vector. The vector can be transferred into a host cell by methods such as transfection, transduction, cell fusion, and lipofection.

The term "bearing" means that another nucleic acid sequence is incorporated into a chromosome or a nucleic acid sequence.

The gene means a nucleotide encoding a polypeptide, and examples thereof include a nucleic acid molecule such as cDNA or genomic DNA.

In the present invention, the Kozak sequence means a sequence before the start codon (ATG) of E4ORF6, and is GCCACC.

In the present invention, the IRES sequence is an internal ribosome entry site. The IRES sequence is not limited as long as the sequence has a function of being recognized by a ribosome without a cap structure on the 5' side of the mRNA and initiating translation. The sequence is preferably a sequence having an identity of 90% or more with the sequence disclosed in seq16. The sequence is more preferably a sequence having an identity of 95% or more with the sequence disclosed in seq16, and most preferably the same sequence as seq16.

<Manufacturing method of adeno-associated virus>

A first aspect of the manufacturing method of an adeno-associated virus of the present invention is a manufacturing method of an adeno-associated virus, including expressing an E4ORF6 gene with an exogenous promoter, in which expression of E4ORF6 is reduced as compared with a case where E4ORF6 is expressed using an expression unit containing an enhancer, a cytomegalovirus-derived promoter, a Kozak sequence, an E4ORF6 gene, and a polyA signal sequence.

A second aspect of the manufacturing method of an adeno-associated virus according to the present invention is a manufacturing method of an adeno-associated virus, including transferring an E4 region containing at least an E4ORF6 gene into a cell, in which an amount of the E4ORF6 gene transferred into a cell is 1/2 or less of the amount of one or more genes transferred into a cell among a Rep gene, a Cap gene, an E2A gene, a VA-RNA gene, or a gene for desired therapy or prophylaxis.

As described above, in the present invention, the adeno-associated virus can be produced in a large amount by reducing the expression level of E4ORF6, which is a gene necessary for the production of the adeno-associated virus. In addition, in the present invention, it is possible to reduce the cytotoxicity by E4ORF6 and enhance the cell viability after gene transfer. Furthermore, in the second aspect of the present invention, since the transfer ratio of the plasmid is reduced, the cost can be reduced.

In the present invention, the E4ORF6 gene is expressed by an exogenous promoter.

The exogenous promoter means a promoter that is not contained in the wild type, and in a case of the E4 gene, it means a promoter that is not contained in the E4 region of the wild-type adeno-associated virus. As the exogenous promoter for expressing the E4ORF6 gene, a cytomegalovirus-derived promoter (CMV promoter) may be used, or a promoter having a weaker transcriptional activity than the CMV promoter may be used. The promoter having a weaker transcriptional activity than the CMV promoter will be described later.

It can be understood by those skilled in the art that a case where E4ORF6 is expressed using an expression unit containing an enhancer, a cytomegalovirus-derived promoter, a Kozak sequence, an E4ORF6 gene, and a polyA signal sequence means that E4ORF6 is strongly expressed. In one aspect, it means a case where E4ORF6 is expressed by seq4. That is, "Expression of E4ORF6 is reduced as compared with a case where E4ORF6 is expressed using an expression unit containing an enhancer, a cytomegalovirus-derived promoter, a Kozak sequence, an E4ORF6 gene, and a polyA signal sequence." in the present invention preferably means that the expression of E4ORF6 is reduced as compared with a case where E4ORF6 is expressed using seq4 (a plasmid having the base sequence set forth in SEQ ID NO: 4) in Examples described later. In addition, in the present invention, a method of expressing the E4ORF6 gene without being mediated by the 5' Cap structure (for example, a method of expressing the E4ORF6 gene using an IRES sequence) is preferable than a method of expressing the E4ORF6 gene mediated by the 5' Cap structure.

In the first aspect of the manufacturing method of an adeno-associated virus according to the present invention, as compared with a case where E4ORF6 is expressed using an expression unit containing an enhancer, a cytomegalovirus-derived promoter, a Kozak sequence, an E4ORF6 gene, and a polyA signal sequence, the expression of E4ORF6 may be reduced, and is reduced to preferably 1/2 or less, more preferably 1/4 or less, still more preferably 1/10 or less, still more preferably 1/50 or less, still more preferably 1/250 or less, and still more preferably 1/1,250 or less.

The reduction of the expression of E4ORF6 is reduction of mRNA expression of E4ORF6 and/or reduction of protein expression of E4ORF6.

As compared with a case where E4ORF6 is expressed using an expression unit containing an enhancer, a cytomegalovirus-derived promoter, a Kozak sequence, an E4ORF6 gene, and a polyA signal sequence, the mRNA expression level of the E4ORF6 gene is preferably 1/2 or less, more preferably 1/4 or less, still more preferably 1/10 or less, still more preferably 1/50 or less, still more preferably 1/250 or less, and still more preferably 1/1,250 or less.

As compared with a case where E4ORF6 is expressed using an expression unit containing an enhancer, a cytomegalovirus-derived promoter, a Kozak sequence, an E4ORF6 gene, and a polyA signal sequence, the expression level of the protein expressed by the E4ORF6 gene is preferably 1/2 or less, more preferably 1/4 or less, still more preferably 1/10 or less, still more preferably 1/50 or less, still more preferably 1/250 or less, and still more preferably 1/1,250 or less.

The means for reducing the expression of E4ORF6 is preferably any one or more of the following (a) to (e). According to the present invention, there is provided an expression vector containing E4ORF6 gene linked to an exogenous promoter, in which the expression vector satisfies any one or more of the following (a) to (e).

(a) the exogenous promoter is a promoter having a weaker transcriptional activity than the cytomegalovirus-derived promoter,
(b) the Kozak sequence of the E4ORF6 gene is a modified Kozak sequence, or the Kozak sequence is not used,
(c) the enhancer sequence is a modified enhancer sequence, or the enhancer sequence is not used,
(d) the polyA signal sequence is a modified polyA signal sequence, or the polyA signal sequence is not used, and
(e) the E4ORF6 gene is expressed using an IRES sequence.

Examples of the promoter having a weaker transcriptional activity than a cytomegalovirus-derived promoter include an EF1α promoter, a CAG promoter, an SV40 promoter, a PGK promoter, an RSV promoter, and the like.

Examples of a method for modifying the Kozak sequence include a method of introducing a mutation into the sequence and a method of reducing the activity by removing the sequence.

Examples of a method for modifying the enhancer sequence include a method of introducing a mutation into the sequence and a method of reducing the activity by removing the sequence.

Examples of a method for modifying the polyA signal sequence include a method of introducing a mutation into the sequence and a method of reducing the activity by removing the sequence.

In the manufacturing method of an adeno-associated virus according to the embodiment of the present invention, the amount of the E4 region transferred into the cell is preferably 1/2 or less, more preferably 1/4 or less, more preferably 1/10 or less, more preferably 1/50 or less, more preferably 1/250 or less, and more preferably 1/1,250 or less of the amount of any one or more genes transferred into the cell among the Rep gene, the Cap gene, the E2A gene, the VA-RNA gene, or the gene for desired therapy or prophylaxis.

In the present invention, a plasmid vector for transferring the E4 region into the cell may be same as or may be different from a plasmid vector for transferring the E2A gene and the VA-RNA gene into the cell.

In the present invention, the E4 region may include an E4ORF6 gene and a gene other than the E4ORF6 gene, or may consist of only an E4ORF6 gene. Preferably, the E4 region consists of only the E4ORF6 gene. Preferably, the E4 region consists of only the E4ORF6 gene and does not include the polyA signal sequence. In addition, the E4 region is preferably present on a downstream of a promoter capable of regulating expression.

In the manufacturing method of an adeno-associated virus according to the embodiment of the present invention, a virus helper gene is transferred into a cell.

The virus helper gene is preferably under the control of a promoter and may be under the control of a promoter capable of regulating expression.

As the promoter capable of regulating expression, a promoter capable of regulating the on and off of expression depending on the presence or absence of a stimulus can be used. Examples of the stimulus include chemical stimuli (intrinsic hormone/stress response, lactose, tetracycline or a derivative thereof (for example, doxycycline), cumic acid, a protein (rapamycin, FKCsA, abscisic acid (ABA), and the like), tamoxifen/Cre-loxP (a system using a Cre promoter modified such that activation can be induced by tamoxifen), and a riboswitch), and a physical stimulus (blue light, heat), but the stimulus is not particularly limited.

The promoter capable of regulating expression may be a promoter capable of regulating expression by a drug. The drug is preferably tetracycline or a derivative thereof (for example, doxycycline).

Examples of the promoter capable of regulating expression include a tetracycline-responsive promoter, a RU486-inductive promoter, an ecdysone-inductive promoter, a rapamycin-inductive promoter, and a metallothionein promoter. Specific examples of the tetracycline-responsive promoter include a Tet-on/off system (manufactured by TET systems GmbH & Co. KG).

The promoter capable of regulating expression may be a Tet-on/off system which is a promoter capable of regulating expression by tetracycline, or a Tet-on system.

In the Tet-on system, the promoter additionally contains at least one Tet operon. By using a Tet operon (tetracycline-controlled transcriptional activation), transcription can be reversibly switched on or off in the presence of one of the tetracycline which is an antibiotic, or a derivative thereof (for example, doxycycline). The Tet repressor protein present in the cell blocks expression by binding to a Tet operator sequence introduced into a promoter. Therefore, no gene expression is observed in a case where the Tet repressor binds to the Tet operator sequence. By adding tetracycline or doxycycline, the Tet repressor is sequestered to allow promoter activity and turn on gene expression. The Tet operon system is widely available, such as the Tet operon used in the pcDNA (trademark) 4/TO mammalian expression vector available from Invitrogen.

In the Tet-on system, the Tet operator sequence may be located upstream or downstream of the promoter for which expression is desired to be regulated. The Tet operator sequence is preferably located downstream of a promoter capable of regulating expression in view of reducing expression leakage in a case where expression is turned off.

The specific examples of the promoter are not particularly limited, but can include a cytomegalovirus-derived promoter (CMV promoter) (including an enhancer as desired), an SV40 early promoter, a human elongation factor-1$\alpha$ (EF-1$\alpha$) promoter, a human ubiquitin C promoter, a retrovirus Rous sarcoma virus (RSV) LTR promoter, a dihydrofolate reductase promoter, a $\beta$-actin promoter, and a phosphoglycerate kinase (PGK) promoter.

The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

The adenovirus refers to a non-enveloped virus having an icosahedron nucleocapsid containing double-stranded DNA in a family of Adenoviridae. Over 50 subtypes of adenovirus have been isolated from humans and many additional subtypes have been isolated from other mammals and birds. These subtypes belong to the family of Adenoviridae and are classified into two genera (that is, Mastadenovirus and Aviadenovirus). These adenoviruses are morphologically and structurally similar. However, in humans, adenoviruses exhibit different immunological properties, and that is, are classified into serum types. Two human serum types of adenovirus (that is, Ad2 and Ad5) have been intensively studied.

The adenovirus-derived virus helper gene is a gene involved in replication and packaging of adeno-associated virus.

Examples of the virus helper gene derived from adenovirus can include E1A, E1B, E2A, E4, and VA-RNA. In the host cell having all or a part of the E1 region, the region of the adenovirus genome required for replicating the AAV genome and packaging of the AAV genome into the capsid to form an AAV virion is the E2A region, the E4 region, and the VA-RNA region. For the function by the E4 region, the 34 kDa E4 protein encoded by the open reading frame 6 (E4ORF6) of the E4 region is required for replicating the AAV. Preferably, the virus helper gene is an E2 gene, an E4 gene (E4ORFG), or a VA-RNA gene. The VA-RNA gene is preferably a VA-RNAI gene.

The adenovirus-derived virus helper gene (such as an E1A, an E1B, an E2A, an E4, and a VA-RNA) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base are made to the wild-type virus helper gene, the number of the modified bases is preferably 1 to 20, more preferably 1 to 10, and still more preferably 1 to 3. The modified base sequence of the virus helper gene exhibits preferably 85% or more sequence identity, more preferably 90% or more sequence identity, still more preferably 95% or more sequence identity, and even still more preferably 98% or more sequence identity with the base sequence of the wild-type virus helper gene.

In a case of transferring a virus helper gene into a cell, a vector containing the virus helper gene can be transferred into the cell.

As the vector containing a virus helper gene, for example, a plasmid, a virus-derived sequence, an artificially designed nucleic acid, or the like can be used, and a plasmid is preferable.

In the manufacturing method of an adeno-associated virus of the present invention, an adeno-associated virus gene is transferred into a cell.

Adeno-associated virus (AAV) refers to a small, incompletely replicative, non-enveloped virus containing single-stranded DNA from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide.

The serum type 1 (AAV1), the serum type 5 (AAV5), and the serum type 6 (AAV6) are serum types having higher tissue directivity. It is said that AAV1 has high gene transfer efficiency into muscle, liver, airway, central nervous system, and the like, AAV5 has high gene transfer efficiency into central nervous system, liver, retina, and the like, and AAV6 has high gene transfer efficiency into heart, muscle, liver, and the like. In the present invention, the serum type 2 or the serum type 5 is preferably used. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

As the adeno-associated virus gene, the Rep gene and the Cap gene are preferably used. The Rep gene and the Cap gene encode proteins involved in the replication and packaging of the virion. In the wild type, the Rep gene is expressed from the P5 promoter and the p19 promoter. The Cap region expresses VP1, VP2, and VP3. Examples of the promoter that is naturally carried by the Cap gene can include a p40 promoter.

The adeno-associated virus gene (such as a Rep gene and a Cap gene) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base are made to the wild-type adeno-associated virus gene (such as a Rep gene and a Cap gene), the number of the modified bases is preferably 1 to 20, more preferably 1 to 10, and still more preferably 1 to 3. The modified base sequence of the adeno-associated virus gene exhibits preferably 85% or more sequence identity, more preferably 90% or more sequence identity, still more preferably 95% or more sequence identity, and even still more preferably 98% or more sequence identity with the base sequence of the wild-type adeno-associated virus gene.

In a case where the Rep gene and the Cap gene are transferred into a cell, a vector containing the Rep gene and the Cap gene can be transferred into the cell. The arrangement of the Rep gene and the Cap gene in a vector is not particularly limited, the Rep gene may be located upstream of the Cap gene or downstream of the Cap gene, and the Rep gene is located preferably upstream of the Cap gene.

The Rep gene means a region of the AAV genome encoding a virus replication protein that is known to those skilled in the art and that is collectively required for replication of a virus genome, or its functional homolog, for example, a human herpesvirus 6 (HHV-6) Rep gene (mediating AAV-2 DNA replication is known). Accordingly, the coding region of the Rep gene includes at least a gene encoding REP78 and REP68 ("long form of REP protein") and REP52 and REP40 ("short form of REP protein") of AAV, or a functional homologue thereof. The coding region of the Rep gene used in the present invention may be derived from any AAV serum type, but is preferably derived from AAV2. Examples of those derived from AAV2 include REP78 and REP68, and REP52 and REP40, and ITR.

The Cap gene means a region in the AAV genome encoding a capsid protein of a virus known to those skilled in the art. Examples of these capsid proteins are AAV capsid proteins VP1, VP2 and VP3. The Cap gene used in the present invention may be derived from any AAV serum type, but is preferably derived from AAV2 or AAV5. AAV5 is particularly preferable.

As the vector containing the Rep gene and the Cap gene, for example, a plasmid, a nucleic acid sequence derived from a virus, or an artificially designed nucleic acid can be used, and a plasmid is preferable.

In the manufacturing method of an adeno-associated virus according to the embodiment of the present invention, a gene for therapy or prophylaxis may be transferred into a cell.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of inflammatory diseases, autoimmunity, chronic and infectious diseases (including disorders such as AIDS, cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The gene for therapy or prophylaxis may be linked to a promoter for expressing the gene for therapy or prophylaxis. The promoter for expressing a gene for treatment or prevention is not particularly limited, but examples thereof can include a cytomegalovirus-derived promoter (including an enhancer as desired), an SV40 early promoter, a human elongation factor-1$\alpha$ (EF-1$\alpha$) promoter, a human ubiquitin C promoter, a retrovirus Rous sarcoma virus LTR promoter, a dihydrofolate reductase promoter, a $\beta$-actin promoter, and a phosphoglycerate kinase (PGK) promoter. The gene for treatment or prevention and the promoter for expressing the gene are preferably flanked by the ITR sequences.

Preferred aspects of the combination of vectors according to the present invention include the following aspects:

(1) an aspect in which a transfer ratio of vector having an E4 gene is reduced by using a vector containing a Rep gene and a Cap gene, a vector containing an E2A gene and a VA-RNA gene, a vector containing an E4 gene, and a vector containing a gene for desired therapy or prophylaxis;

(2) an aspect in which a transfer ratio of vector having an E4ORF6 gene is reduced by using a vector containing a Rep gene and a Cap gene, a vector containing an E2A gene and a VA-RNA gene, a vector containing an E4ORF6 gene, and a vector containing a gene for desired therapy or prophylaxis;

(3) an aspect in which a vector containing a Rep gene and a Cap gene, a vector containing an E2A gene and a VA-RNA gene, a vector containing an E4ORF6 gene and having a decreased expression level of the E4ORF6 gene, and a vector containing a gene for desired therapy or prophylaxis are used;

(4) an aspect in which a vector containing a Rep gene and a Cap gene, a vector containing an E2A gene, an E4ORF6 gene, and a VA-RNA gene and having a decreased expression level of the E4ORF6 gene, and a vector containing a gene for desired therapy or prophylaxis are used;

(5) an aspect in which a transfer ratio of vector having an E4 gene is reduced by using a vector containing a Rep gene, a Cap gene, an E2A gene, and a VA-RNA gene, a vector containing an E4 gene, and a vector containing a gene for desired therapy or prophylaxis;

(6) an aspect in which a vector containing a Rep gene, a Cap gene, an E2A gene, and a VA-RNA gene, a vector containing an E4ORF6 gene and having a decreased expression level of the E4ORF6 gene, and a vector containing a gene for desired therapy or prophylaxis are used;

(7) an aspect in which a transfer ratio of vector having an E4 gene is reduced by using a vector containing a Rep gene, a Cap gene, an E2A gene, a VA-RNA gene, and a gene for desired therapy or prophylaxis, and a vector containing an E4 gene;

(8) an aspect in which a vector containing a Rep gene, a Cap gene, an E2A gene, a VA-RNA gene, and a gene for desired therapy or prophylaxis, and a vector containing E4ORF6 gene and having a decreased expression level of the E4ORF6 gene are used;

(9) an aspect in which a vector containing a Rep gene, a Cap gene, an E2A gene, an E4ORF6 gene, and a VA-RNA gene and having a decreased expression level of the E4ORF6 gene, and a vector containing a gene for desired therapy or prophylaxis are used;

(10) an aspect in which a vector containing a Rep gene, a Cap gene, an E2A gene, an E4ORF6 gene, a VA-RNA gene, and a gene for desired therapy or prophylaxis and having a decreased expression level of the E4ORF6 gene are used.

In the manufacturing method of an adeno-associated virus according to the embodiment of the present invention, an adeno-associated virus can be manufactured by culturing a cell containing a virus helper gene, an adeno-associated virus gene, and, if necessary, a gene for desired therapy or prophylaxis.

As a manufacturing method of the adeno-associated virus, any of a triple transfection (TT) method, a packaging cell method, or a producer cell method may be used. In a case where E4ORF6 is used instead of the E4 gene, the TT method is particularly preferable.

The TT method is a method in which a plasmid containing a Rep gene and a Cap gene, an adenovirus helper plasmid, and a plasmid containing a transgene (for example, a gene for desired therapy or prophylaxis) are simultaneously transfected into HEK293 cells, and can thus be packaged as recombinant AAV (rAAV).

The packaging cell method is a method of producing AAV by transferring, to the cell in which a part of the gene in the TT method is incorporated into a chromosome in advance, the remaining genes into a cell by transfection.

The producer cell method is a method of producing AAV without a transfection operation, using the cell in which all genes in the TT method are incorporated into chromosomes.

Particularly preferred examples of an aspect of the present invention can include the following aspects.

A manufacturing method of an AAV gene, including a step of performing gene transfer of a plasmid bearing a Rep gene and a Cap gene, a plasmid bearing an E2A gene, a VA-RNA gene, and an E4ORF6, and a plasmid bearing a GOI gene, in which the plasmid bearing the E4ORF6 does not have an enhancer for causing the E4ORF6 to be expressed and a polyA signal sequence.

A manufacturing method of an AAV gene, including a step of performing gene transfer of a plasmid bearing a Rep gene and a Cap gene, a plasmid bearing an E2A gene, a VA-RNA gene, and E4ORF6, and a plasmid bearing a GOI gene, in which a promoter that causes E4ORF6 of the plasmid bearing E4ORF6 to be expressed is a CAG promoter.

The cell culture in the manufacturing method of an adeno-associated virus according to the embodiment of the present invention can be carried out under normal conditions for cell culture. The culture medium to be used and the culture conditions can be appropriately selected by those skilled in the art. As the culture medium, the medium includes Expi29 Expression Medium (Thermo Fisher Scientific, A1435101), a Dulbecco's Modified Eagle's Medium (DMEM) containing 10% (vol/vol) bovine fetal serum (FBS), and a serum-free UltraCULTURE (trademark) medium (Lonza) can be used, but the present invention is not particularly limited.

The culture temperature is generally 25°C to 45°C, preferably 30°C to 42°C, more preferably 35°C to 40°C, and 37°C as an example.

The $CO_2$ concentration is generally 3 to 10% $CO_2$, preferably 5 to 10% $CO_2$, and 8% $CO_2$ as an example.

The cells can be cultured in a culture medium of any volume, for example, the cells can be cultured in a culture medium of 1 mL to 2,000 L, preferably 1 L to 2,000 L, more preferably 50 L to 2,000 L, and particularly preferably 500 L to 2,000 L.

The culture may be carried out while stirring by shaking. The stirring speed in the case of stirring by shaking is generally 50 rpm to 200 rpm, and preferably 80 to 150 rpm.

The stirring culture may be rotary stirring culture using a propeller or the like in a reactor. The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

The culture time in each step is not particularly limited, but is generally 6 hours to 14 days, preferably 12 hours to 7 days, more preferably 24 hours to 144 hours, and still more preferably 24 hours to 96 hours.

The titer of the manufactured adeno-associated virus can be measured by a conventional method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding $MgCl_2$ and Benzonase to the collected supernatant to react. It is possible to measure the titer of AAV by performing ddPCR (Droplet Digital PCR) using the sample containing the adeno-associated virus obtained above as a ddPCR sample.

<Cell>

A first aspect of the cell according to the present invention is a cell containing at least an E4ORF6 gene to be expressed by an exogenous promoter, in which expression of E4ORF6 is reduced as compared with a case where E4ORF6 is expressed using an expression unit containing an enhancer, a cytomegalovirus-derived promoter, a Kozak sequence, an E4ORF6 gene, and a polyA signal sequence.

In the cell of the present invention, preferably, the E4ORF6 gene expressed by the exogenous promoter is transferred into the cell by an expression vector satisfying any one or more of the following (a) to (e):

(a) the exogenous promoter is a promoter having a weaker transcriptional activity than the cytomegalovirus-derived promoter,
(b) the Kozak sequence of the E4ORF6 gene is a modified Kozak sequence, or a Kozak sequence is not provided,

(c) the enhancer sequence is a modified enhancer sequence, or the enhancer sequence is not provided,

(d) the polyA signal sequence is a modified polyA signal sequence, or the polyA signal sequence is not provided, and

(e) the E4ORF6 gene is expressed using an IRES sequence.

A second aspect of the cell according to the present invention is a cell comprising at least an E4ORF6 gene to be expressed by an exogenous promoter, in which an amount of the E4ORF6 gene transferred into a cell is lower than an amount of the one or more genes transferred into the cell among a Rep gene, a Cap gene, an E2A gene, a VA-RNA gene, or a gene for desired therapy or prophylaxis.

The cell according to the embodiment of the present invention may further have an E2A gene and a VA-RNA gene.

The cell according to the embodiment of the present invention may further have a Rep gene.

The cell according to the embodiment of the present invention may further have a Cap gene and/or a gene for desired therapy or prophylaxis.

The details of each of the components in the cell according to the embodiment of the present invention are as described above in the present specification.

The cell is preferably a eukaryotic cell, and is, for example, a mammalian cell or an insect cell. The cell is more preferably a mammalian cell. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK Jurkat, NIH3T3, PC12, a baby hamster kidney (BHK) cell, VERO, SP2/0, YB2/0, Y0, C127, an L cell, COS (for example, COS1 and COS7), QC1-3, a human embryoderived kidney (HEK293) cell, VERO, PER. C6, HeLa, EB1, EB2, EB3, and oncolytic or hybridoma cell lines. The cell is preferably a HEK293 cell or a CHO cell, and more preferably a HEK293 cell.

The cell according to the embodiment of the present invention has at least the E4ORF6 gene expressed by the exogenous promoter. Preferably, the vector containing the E4ORF6 gene is contained in a chromosome. Being contained in a chromosome means that the whole length or a part of the vector is integrated into the chromosome. Since the vector is integrated into the chromosome, the gene required for the production of the adeno-associated virus is constitutively maintained in the cell, and the adeno-associated virus can be produced at an optional timing.

The vector containing the E4ORF6 gene may be transferred to be expressed, but it is more preferable that the expression is permanent. Permanent expression means that in a case where the cell is divided, the vector containing the E4ORF6 gene is also replicated, and the cell after the division also has the vector. Permanent expression can be achieved by integrating the vector into the chromosome of the cell or by using a vector capable of replicating in the cytoplasm.

The vector containing the E4ORF6 gene can be transferred into a cell by methods such as transfection, transduction, and lipofection.

Transfection refers to transfer of a nucleic acid into a cell across a membrane of eukaryotic cells by chemical means (for example, calcium phosphate mediated precipitation), mechanical means (for example, electroporation), or physical means (for example, delivery by bioballistic).

Transduction refers to transfer of a nucleic acid into a cell across a membrane of a eukaryotic cell via a virus-derived vector.

Lipofection refers to transfer of a nucleic acid into a cell by the formation of a complex between a vector and a positively charged lipid or the like by electrical interaction, and endocytosis or membrane fusion.

The present invention will be described in more detail with reference to the following examples, but the present invention is not limited to the examples.

Examples

[Materials and methods]

<Cell culture>

Floating HEK293 cells (Thermo Fisher Scientific, R79007) were suspended in 12 mL of Expi29 Expression Medium (Thermo Fisher Scientific, A1435101) in a $1 \times 10^7$ cells/mL, and cultured in 125 mL shaking flask for 24 hours. The culture solution was incubated under the conditions of 37°C and 8% $CO_2$ while being constantly stirred at 120 rpm.

<Plasmid>

AAVpro (register trademark) Helper Free System (AAV5) (Takara Bio Inc., 6650) was used (seq1, seq2). The entire sequences of the used plasmids (seq1 to seq7) are set forth in SEQ ID NOs: 1 to 7 in the sequence list. The sequences of seq14 to seq16 are set forth in SEQ ID NOs: 14 to 16 in the sequence list.

seq1 (GOI): a GOI-bearing plasmid
seq2 (RC): a plasmid bearing a Rep gene and a Cap gene
seq3 (ΔE4): a plasmid in which the E4 gene coding region was removed based on pHelper (Takara Bio) and then re-circularized by self-ligation
seq4 (CMV-E4ORF6): a plasmid (GENEWIZ) in which a CMV-E4ORF6 gene sequence was synthesized and inserted into an EcoRV site of a pUC57 vector

seq5 (E4): a plasmid (GENEWIZ) obtained by synthesizing an E4 region sequence and inserting the sequence into the EcoRV site of a pUC57 vector

seq6 (CAG-E4ORF6): a plasmid in which the E4ORF6 gene sequence was inserted on the downstream of the CAG promoter

seq7 (CMV-E4ORF6ΔEnhancerΔPolyA): a plasmid in which the Enhancer sequence and the PolyA signal sequence were removed from seq4

seql4 (TFT-RFP-IRFS-E4ORF6): a plasmid in which an E4ORF6 gene is connected to the downstream of a red fluorescent protein (RFP) gene under a promoter capable of regulating expression where Tet operator sequence is incorporated, through IRES

seq15 (TET-RFP-IRES-E4ORF6ΔPolyA): a plasmid in which polyA is removed from seq8

seql6: IRES sequence

<Real-time PCR>

The HEK cells cultured 72 hours after the gene transfer under the conditions of 37°C and 8% $CO_2$ were collected by centrifugation at 300 x g. mRNA was purified using RNeasy Plus Mini Kit (Qiagen), and cDNA was prepared by reverse transcription using High Capacity RNA to cDNA Kit. The prepared cDNA was quantitatively evaluated by detection of E4ORF6 with primers (seq8 and seq9) and a probe (seq10) for E4ORF6 using Taq-Man Gene Expression Master Mix (Applied Biosystems), and by the normalization using TaqMan Gene Expression Assay for GAPDH (Thermo Fisher Scientific, Hs02786624_g1). Sequences of seq8 to seq10 are set forth in SEQ ID NOs: 8 to 10 in the sequence list.

<Measurement of AAV titer>

The HEK cell culture solution, which had been cultured for 72 hours after the gene transfer under the conditions of 37°C and 8% $CO_2$, was collected, and the cells were disrupted by freezing and thawing at -80°C. Then, the supernatant was collected by centrifugation at 13,800 ×g, 2 mmol/L (final concentration) $MgCl_2$, and 25 U/mL Benzonase was added thereto, and a reaction was carried out at 37°C for 2 hours to digest gDNA (genomic DNA) and residual plasmid. The sample after the reaction was incubated at 95°C for 15 minutes, 70°C for 3 minutes, 40°C for 3 minutes, and 20°C for 3 minutes in this order, to inactivate Benzonase, and the reactant was used as a ddPCR (Droplet Digital PCR) sample. The ddPCR sample was diluted 50-fold with a solution in which 0.05% Pluronic F-68 and 10 μg/mL bovine thymus DNA was mixed to a TE buffer (pH 8.0). 10 μL of ddPCRtm Supermix for Probes (no dUTP) (Bio-Rad Laboratories, Inc.), 2 μL of diluted ddPCR sample, primers (seq11, seq12, final concentration of 900 nmol/L) and probe (seq13, final concentration of 250 nmol/L), nuclease free water (Thermo Fisher Scientific, 10977015) was mixed in a tube on ice, the total liquid volume was adjusted to 22 μL, and ddPCR was performed. The sequences of seq11, seq12, and seq13 are set forth in SEQ ID NOs: 11 to 13 in the sequence list.

In ddPCR, droplets were formed from the prepared solution using a droplet forming apparatus (QX200 Droplet Generator, BIORAD), and subsequently incubated at 95°C for 10 minutes, 94°C for 30 seconds, and 55°C in this order for 40 cycles, and then incubated at 98°C for 10 minutes. The measurement was carried out using a droplet reader (QX200 Droplet Reader, BIO-RAD), and the AAV genome titer (vg/mL) was calculated.

<Evaluation of cytotoxicity>

As the toxicity of the cell in which the gene was transferred, the rate of the cytotoxicity (%) was calculated by performing trypan blue staining 72 hours after the gene transfer and counting the number of stained positive cells.

[Result]

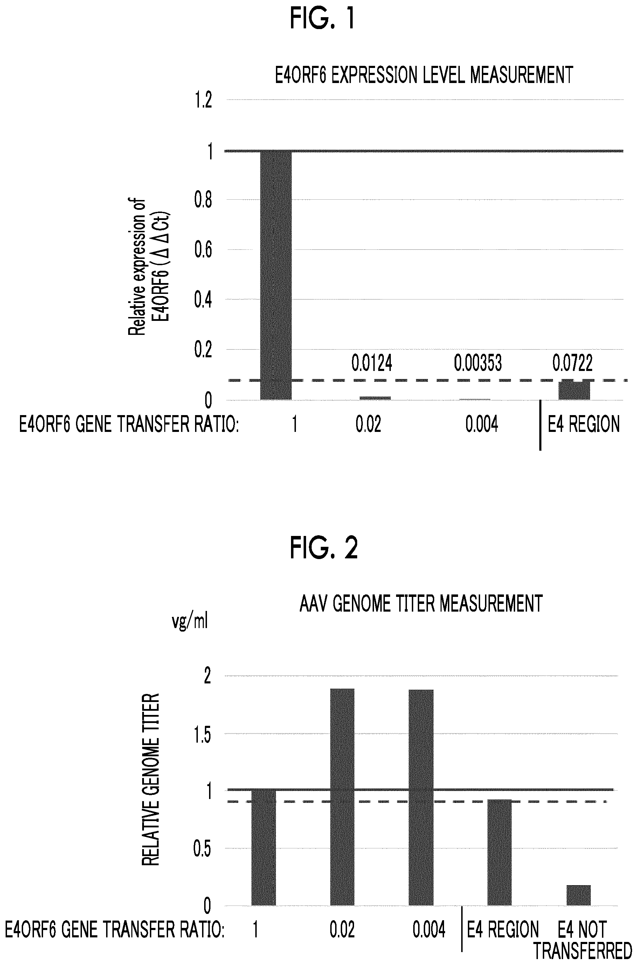

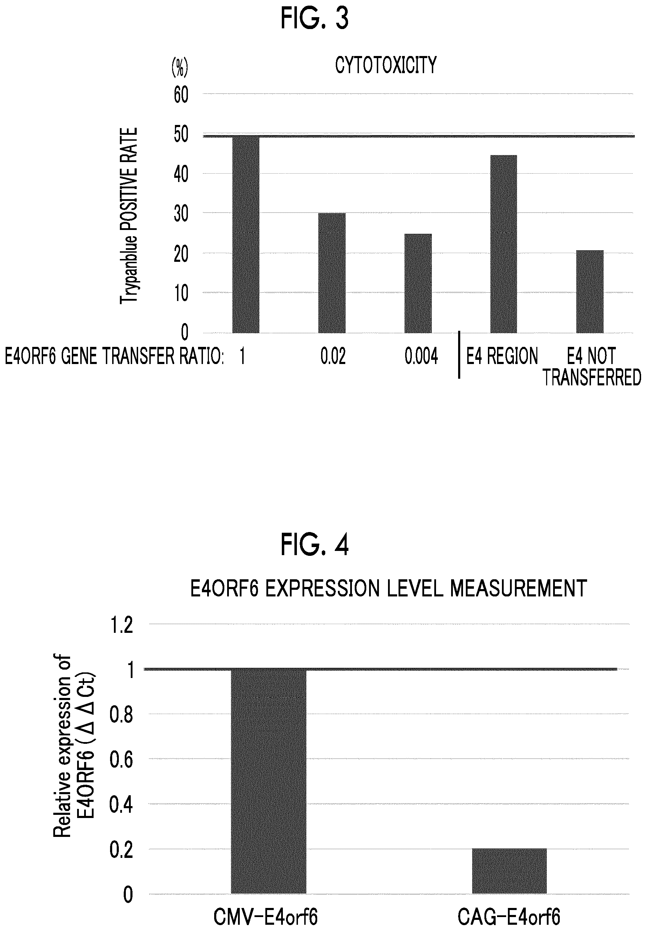

The change in the production amount of AAV in a case where the amount of the plasmid bearing E4ORF6 to be transferred was changed, was evaluated. Fig. 1 shows the results of quantitatively evaluating the E4ORF6 expression level 72 hours after transferring a gene into a HEK293 cell in the following combinations, Fig. 2 shows the results of measuring AAV titer by ddPCR, and Fig. 3 shows the rate of cytotoxicity by trypan blue staining.

E4ORF6 gene transfer ratio 1

$$seq1:seq2:seq3:seq4 = 1:1:1:1$$

E4ORF6 gene transfer ratio 0.02

$$seq1:seq2:seq3:seq4 = 1:1:1:0.02$$

E4ORF6 gene transfer ratio 0.004

$$seq1:seq2:seq3:seq4 = 1:1:1:0.004$$

E4 gene

$$seq1:seq2:seq3:seq5 = 1:1:1:1$$

E4: not transferred

$$seq1:seq2:seq3:seq5 = 1:1:1:0$$

E4ORF6 expression level

A case where E4ORF6 gene transfer ratio is 1:1, is set as a reference (1).
E4ORF6 gene transfer ratio 0.02:0.0124
E4ORF6 gene transfer ratio 0.004:0.000353
E4 gene: 0.0722

Virus amount (vg/mL)

A case where E4ORF6 gene transfer ratio is 1:1, is set as a reference (1).
E4ORF6 gene transfer ratio 0.02:1.90
E4ORF6 gene transfer ratio 0.004:1.88
E4 gene: 0.924
E4 not transferred: 0.179

Cytotoxicity (%)

E4ORF6 gene transfer ratio 1:49.0
E4ORF6 gene transfer ratio 0.02:29.9
E4ORF6 gene transfer ratio 0.004:24.9
E4 gene: 44.6
E4 not transferred: 20.6

From the results in Fig. 1, it was found that the transfer ratio of the plasmid encoding the E4ORF6 gene is reduced to reduce the expression level of E4ORF6.

From the results in Fig. 2, it was found that the AAV production amount was improved by reducing the transfer ratio of the plasmid.

From the results in Fig. 3, it was found that the cytotoxicity was reduced by reducing the transfer ratio of the plasmid.

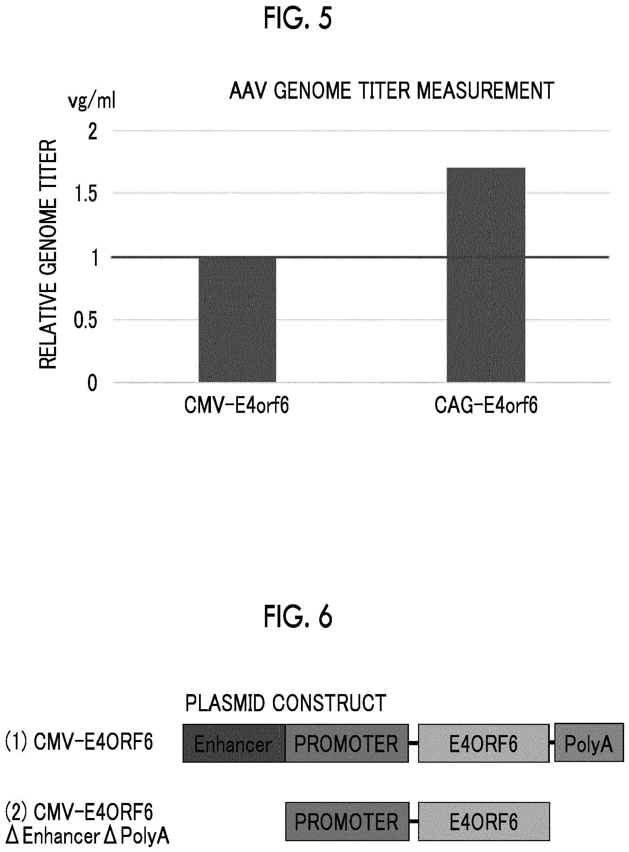

The change in the AAV production amount in a case where the expression promoter of E4ORF6 was changed was evaluated. Fig. 4 shows the results of quantitatively evaluating the E4ORF6 expression level 72 hours after transferring a gene into a HEK293 cell in the following combinations, and Fig. 5 shows the results of measuring AAV titer by ddPCR.

CMV-E4ORF6

$$seq1:seq2:seq3:seq4 = 1:1:1:1$$

CAG-E4ORF6:

$$seq1:seq2:seq3:seq6 = 1:1:1:1$$

E4ORF6 expression level

A case of CMV-E4ORF6 is set as a reference (1).
CAG-E4ORF6: 0.203

Virus amount (vg/mL)

A case of CMV-E4ORF6 is set as a reference (1).
CAG-E4ORF6: 1.70

From the results in Fig. 4, it was found that the expression level of E4ORF6 is reduced by changing the promoter of the plasmid encoding the E4ORF6 gene from CMV to CAG.

From the results in Fig. 5, it was found that the AAV production amount was improved by changing the promoter.

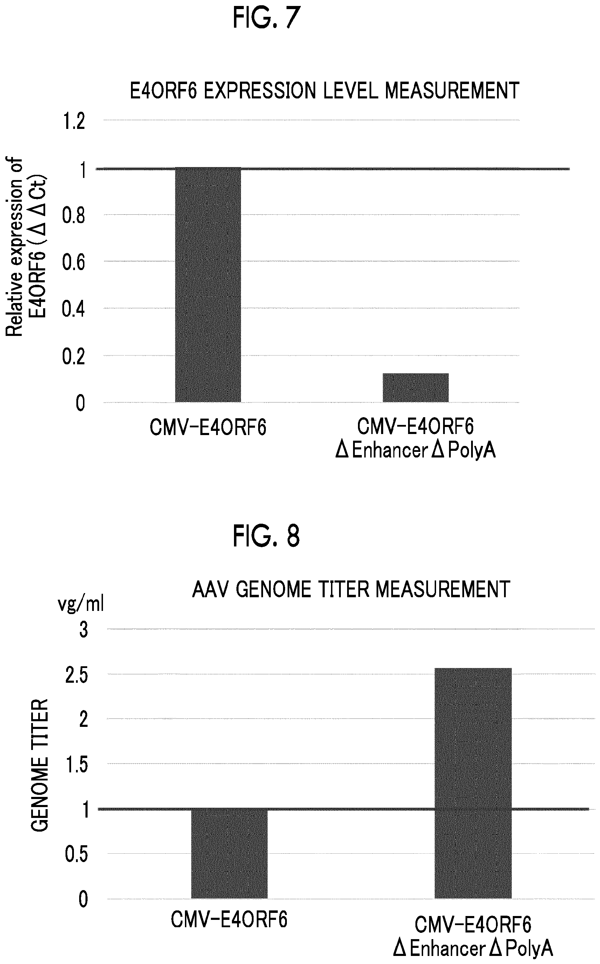

The change in the AAV production amount in a case where the expression regulatory sequence of E4ORF6 was changed, was evaluated. Fig. 6 shows an E4ORF6 plasmid diagram in which the expression regulatory sequence has been changed. Fig. 7 shows the results of quantitatively evaluating the E4ORF6 expression level 72 hours after transferring a gene into a HEK293 cell in the following combinations, and Fig. 8 shows the results of measuring AAV titer by ddPCR.

CMV-E4ORF6

$$seq1:seq2:seq3:seq4 = 1:1:1:1$$

CMV-E4ORF6ΔEnhancerΔPolyA:

$$seq1:seq2:seq3:seq7 = 1:1:1:1$$

E4ORF6 expression level

A case of CMV-E4ORF6 is set as a reference (1).
CMV-E4ORF6ΔEnhancerΔPolyA: 0.121

Virus amount (vg/mL)

A case of CMV-E4ORF6 is set as a reference (1).
CMV-E4ORF6ΔEnhancerΔPolyA: 2.57

From the results in Fig. 7, it was found that the expression level of E4ORF6 was reduced by removing the enhancer and the polyA signal sequence from the plasmid encoding the E4ORF6 gene. From the results in Fig. 8, it was found that the AAV production amount was improved by the sequence change.

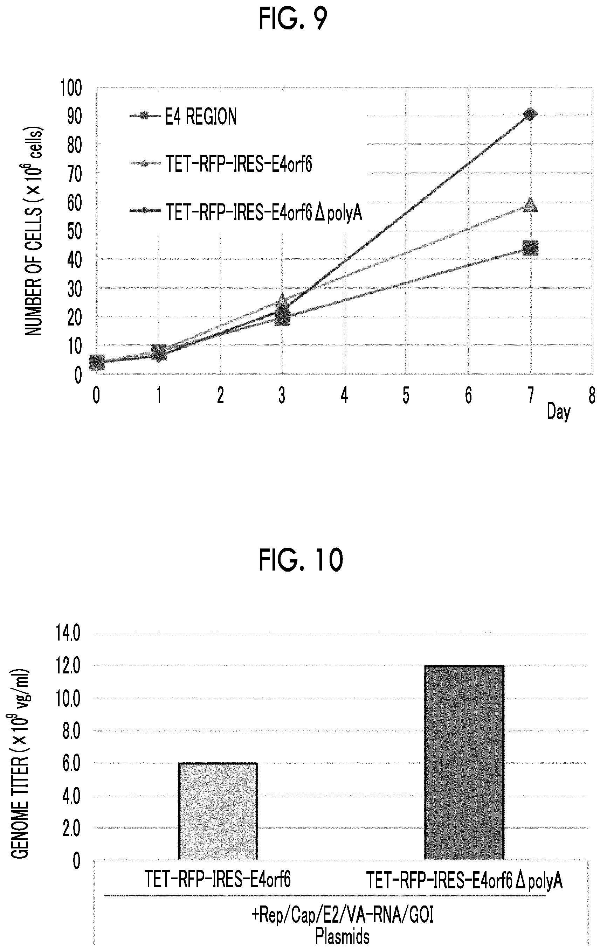

Each of the plasmids of the design (seql4: TET-RFP-TRES-E4ORF6) in which an E4ORF6 gene is connected to the downstream of a red fluorescent protein (RFP) gene under a promoter capable of regulating expression where Tet operator sequence is incorporated, through IRES and the design (seq15: TET-RFP-IRES-E4ORF6ΔPolyA) in which a polyA signal sequence downstream of the E4ORF6 gene was removed was transferred into a HEK cell, and the increase of number of cells by proliferation in the absence of tetracycline was measured. The increase in the number of cells into which the E4 region (seq5) was transferred was measured under the same conditions, and the results of the comparison are shown in Fig. 9.

From the results in Fig. 9, it was found that the number of cells of the seq14 and seq15 transferred cells was larger than that of the E4 region transferred cells.

TET-RFP-IRES-E4ORF6 and TET-RFP-IRES-E4ORF6ΔPolyA were transferred into the HEK cell to prepare a stable expression cell into which a plasmid was incorporated by the treatment with the G418 drug. Fig. 10 shows the results of transferring a Rep gene, a Cap gene, an E2 gene, and a VA-RNA gene into the stable expression cell and measuring the AAV production titer.

From the results in Fig. 10, it was confirmed that AAV can be produced using the prepared stable expression cells of TET-RFP-IRES-E4ORF6 and TET-RFP-IRES-E4ORF6ΔPolyA. In addition, it was found that the titer was improved by removing the PolyA signal sequence.

[Sequence list] International application 21F01312W1JP22036684_0.xml based on International Patent Cooperation Treaty

**Claims**

1. A manufacturing method of an adeno-associated virus, comprising:

   expressing an E4ORF6 gene with an exogenous promoter,
   wherein expression of E4ORF6 is reduced as compared with a case where E4ORF6 is expressed using an expression unit containing an enhancer, a cytomegalovirus-derived promoter, a Kozak sequence, an E4ORF6 gene, and a polyA signal sequence.

2. The manufacturing method of an adeno-associated virus according to claim 1,
   wherein the reduction of the expression of E4ORF6 is reduction of mRNA expression of E4ORF6 and/or reduction of protein expression of E4ORF6.

3. The manufacturing method of an adeno-associated virus according to claim 1 or 2,
   wherein expression level of E4ORF6 is reduced to 1/2 or less as compared with a case where E4ORF6 is expressed using an expression unit containing an enhancer, a cytomegalovirus-derived promoter, a Kozak sequence, an E4ORF6 gene, and a polyA sequence.

4. The manufacturing method of an adeno-associated virus according to any one of claims 1 to 3,
   wherein the expression of E4ORF6 is reduced by any one or more of the following (a) to (e),

   (a) the exogenous promoter is a promoter having a weaker transcriptional activity than the cytomegalovirus-derived promoter,
   (b) the Kozak sequence of the E4ORF6 gene is a modified Kozak sequence, or the Kozak sequence is not used,
   (c) the enhancer sequence is a modified enhancer sequence, or the enhancer sequence is not used,
   (d) the polyA signal sequence is a modified polyA signal sequence, or the polyA signal sequence is not used, and
   (e) the E4ORF6 gene is expressed using an IRES sequence.

5. The manufacturing method of an adeno-associated virus according to any one of claims 1 to 4,
   wherein an amount of the E4ORF6 gene transferred into a cell is 1/2 or less of an amount of one or more genes transferred into a cell among a Rep gene, a Cap gene, an E2A gene, a VA-RNA gene, or a gene for desired therapy or prophylaxis.

6. A manufacturing method of an adeno-associated virus, comprising:

   transferring an E4 region containing at least an E4ORF6 gene into a cell,
   wherein an amount of the E4ORF6 gene transferred into the cell is 1/2 or less of an amount of one or more genes transferred into the cell among a Rep gene, a Cap gene, an E2A gene, a VA-RNA gene, or a gene for desired therapy or prophylaxis.

7. The manufacturing method of an adeno-associated virus according to claim 6,
   wherein an amount of the E4 region transferred into the cell is 1/10 or less of the amount of the one or more genes transferred into the cell among the Rep gene, the Cap gene, the E2A gene, the VA-RNA gene, or the gene for desired therapy or prophylaxis.

8. The manufacturing method of an adeno-associated virus according to claim 6 or 7,
   wherein a plasmid vector for transferring the E4 region into the cell is different from a plasmid vector for transferring the E2A gene and the VA-RNA gene into the cell.

9. The manufacturing method of an adeno-associated virus according to any one of claims 6 to 8,
   wherein the E4 region consists of only the E4ORF6 gene.

10. A cell comprising:

    at least an E4ORF6 gene to be expressed by an exogenous promoter,

wherein expression of E4ORF6 is reduced as compared with a case where E4ORF6 is expressed using an expression unit containing an enhancer, a cytomegalovirus-derived promoter, a Kozak sequence, an E4ORF6 gene, and a polyA signal sequence.

11. The cell according to claim 10,
wherein the E4ORF6 gene expressed by the exogenous promoter is transferred into the cell by an expression vector which satisfies any one or more of the following (a) to (e),

   (a) the exogenous promoter is a promoter having a weaker transcriptional activity than the cytomegalovirus-derived promoter,
   (b) the Kozak sequence of the E4ORF6 gene is a modified Kozak sequence, or a Kozak sequence is not provided,
   (c) the enhancer sequence is a modified enhancer sequence, or the enhancer sequence is not provided,
   (d) the polyA signal sequence is a modified polyA signal sequence, or the polyA signal sequence is not provided, and
   (e) the E4ORF6 gene is expressed using an IRES sequence.

12. A cell comprising:

   at least an E4ORF6 gene to be expressed by an exogenous promoter,
   wherein an amount of the E4ORF6 gene transferred into a cell is lower than an amount of one or more genes transferred into the cell among a Rep gene, a Cap gene, an E2A gene, a VA-RNA gene, or a gene for desired therapy or prophylaxis.

13. The cell according to any one of claims 10 to 12, further comprising:

   an E2A gene; and
   a VA-RNA gene.

14. The cell according to any one of claims 10 to 13, further comprising:
a Rep gene.

15. The cell according to any one of claims 10 to 14, further comprising:

   a Cap gene; and/or
   a gene for desired therapy or prophylaxis.

16. An expression vector comprising:

   E4ORF6 gene linked to an exogenous promoter,
wherein the expression vector satisfies any one or more of the following (a) to (e),

   (a) the exogenous promoter is a promoter having a weaker transcriptional activity than the cytomegalovirus-derived promoter,
   (b) a Kozak sequence of the E4ORF6 gene is a modified Kozak sequence, or the Kozak sequence is not provided,
   (c) an enhancer sequence is a modified enhancer sequence, or the enhancer sequence is not provided,
   (d) a polyA signal sequence is a modified polyA signal sequence, or the polyA signal sequence is not provided, and
   (e) the E4ORF6 gene is expressed using an IRES sequence.

# FIG. 1

E4ORF6 EXPRESSION LEVEL MEASUREMENT

# FIG. 2

AAV GENOME TITER MEASUREMENT

# FIG. 3

CYTOTOXICITY

(%)

Trypanblue POSITIVE RATE

E4ORF6 GENE TRANSFER RATIO: 1      0.02      0.004     | E4 REGION     E4 NOT TRANSFERRED

# FIG. 4

E4ORF6 EXPRESSION LEVEL MEASUREMENT

Relative expression of E4ORF6 ($\Delta \Delta$Ct)

CMV-E4orf6                CAG-E4orf6

## FIG. 5

AAV GENOME TITER MEASUREMENT

## FIG. 6

PLASMID CONSTRUCT

(1) CMV-E4ORF6

(2) CMV-E4ORF6
Δ Enhancer Δ PolyA

## FIG. 7

E4ORF6 EXPRESSION LEVEL MEASUREMENT

## FIG. 8

AAV GENOME TITER MEASUREMENT

## FIG. 9

## FIG. 10

## INTERNATIONAL SEARCH REPORT

| International application No. |
|---|
| **PCT/JP2022/036684** |

**A. CLASSIFICATION OF SUBJECT MATTER**

*C12N 7/01*(2006.01)i; *C12N 5/10*(2006.01)i; *C12N 15/86*(2006.01)i; *C12N 15/864*(2006.01)i
FI:   C12N7/01 ZNA; C12N5/10; C12N15/86 Z; C12N15/864 100Z

According to International Patent Classification (IPC) or to both national classification and IPC

**B. FIELDS SEARCHED**

Minimum documentation searched (classification system followed by classification symbols)

C12N7/01; C12N5/10; C12N15/86; C12N15/864

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2008-526188 A (HOLM, PER SONNE) 24 July 2008 (2008-07-24) claims, paragraphs [0312], [0408], [0463], [0609]-[0612] | 1-4, 10-11, 16 |
| Y | ALLEN, J. M. et al., Improved adeno-associated virus vector production with transfection of a single helper adenovirus gene, E4orf6, MOLECULAR THERAPY (2000) vol. 1, no. 1, pp. 88-95, ISSN:1525-0016 in particular, abstract, p. 89, right column, paragraphs 2, 3, fig. 1-5, table 1 | 1-16 |
| Y | NAYAK, R. et al., Positive and negative effects of adenovirus type 5 helper functions on adeno-associated virus type 5 (AAV5) protein accumulation govern AAV5 virus production, JOURNAL OF VIROLOGY (2007) vol. 81, no. 5, pp. 2205-2212, ISSN:0022-538X in particular, abstract, p. 2210, left column, paragraph 2 to p. 2211, left column, paragraph 1, fig. 4-6 | 1-16 |
| A | LIU, Z. et al., A systematic comparison of relative promoter/enhancer activities in mammalian cell lines, ANALYTICAL BIOCHEMISTRY (1997) vol. 246, pp. 150-152 , ISSN:0003-2697 entire text, all drawings, in particular, fig. 2 | 1-16 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 December 2022** | **13 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
|---|
| **PCT/JP2022/036684** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "The form of Annex C/ST.25 text file" above shall read as "the form of ST.26.".

Form PCT/ISA/210 (continuation of first sheet) (January 2015)

INTERNATIONAL SEARCH REPORT
Information on patent family members

International application No.

**PCT/JP2022/036684**

| Patent document cited in search report | | | Publication date (day/month/year) | Patent family member(s) | | | Publication date (day/month/year) |
|---|---|---|---|---|---|---|---|
| JP | 2008-526188 | A | 24 July 2008 | US | 2010/0310554 | A1 | |
| | | | | claims, paragraphs [0299], [0459], [0568], [0713]-[0716] | | | |
| | | | | EP | 1831382 | A2 | |
| | | | | WO | 2006/070023 | A2 | |

Form PCT/ISA/210 (patent family annex) (January 2015)

22

**REFERENCES CITED IN THE DESCRIPTION**

**Patent documents cited in the description**

- JP 2020188763 A
- JP 2020507331 A
- WO 2020208379 A
- US 20160222356 A